# EUROPEAN PATENT APPLICATION

(11) **EP 1 157 656 A1**
(43) Date of publication of application: **28.11.2001**
(21) Application number: 01111999.7
(22) Date of filing: 22.05.2001
(51) Int. Cl.: A61B 3/04, A61B 3/09

(54) **Device for subjective measurement of ocular convergence**

(30) Priority: 26.05.2000 IT PD000142
(71) Applicant: De Lorenzo, Dario, 35027 Noventa Padovana, (Prov. of Padova) (IT)
(72) Inventor: De Lorenzo, Dario, 35027 Noventa Padovana, (Prov. of Padova) (IT)
(74) Representative: Modiano, Guido, Dr.-Ing.

(57) **Abstract**

The present invention relates to a device particularly for measuring subjective ocular convergence.

The device (10) is characterized in that it comprises a device frame, which can be coupled to the frame of a pair of eyeglasses (12), and supports two elements (15) which can each be subjected to a horizontal translational motion by the user at a lens on a plane which is parallel to the plane of arrangement of the lenses, each one of the movable elements (15) comprising: a first portion for kinematic connection to the device frame; a second portion (21), which corresponds to the upper portion of the lens and is provided with at least one row of first reference marks, for the corresponding pupil of the user, the marks being arranged in vertical succession; and a third portion, which corresponds to the lower portion of the lens, being provided with a second reference mark for the corresponding pupil of the user, and suitable to form a sight. The translational motions of the movable elements (15) can be detected by means of a graduated scale (20) of the device frame.

## Description

The present invention relates to a device particularly for measuring subjective ocular convergence.

The use of progressive-addition or bifocal lenses is currently increasingly widespread; these lenses allow their wearer, affected for example by myopia or hypermetropia problems, to see clearly both at a distance and at close range.

Progressive-addition lenses are substantially constituted by an upper portion whose power allows the user clear distance vision and by a lower portion whose central corridor allows the user clearly near vision.

The lens portions that lie to the side of the central corridor in the lower part of the lens are regions which are not graduated correctly and through which the eye of the user has blurred vision.

In practice, when the eye of the user is gazing at infinity, the pupil sees through the upper portion of the lens, which corrects the user's sight appropriately.

When instead the user looks at objects located at close range, for example in order to read, the pupil moves according to a certain convergence, and in order to allow clear vision the user's sight should be mediated by the graduated central corridor.

It is evident that the position of the central corridor is very important.

In fact, if the vision of close objects is not mediated exactly by the central corridor, vision is unclear and blurred.

The currently known method for producing progressive-addition lenses consists in measuring the so-called interpupillary distance range of the person and then determining the so-called standard convergence by following predefined parameters.

Convergence is defined as the movement that the pupil performs when it looks at a near object after looking at a distant one.

Convergence therefore changes for each person, and for this very reason multifocal lenses produced according to standard criteria are quite often unsuitable for some persons whose pupils, when looking at near objects, do not see through the central corridor of the lower portion but through one of the two lateral portions.

The aim of the present invention is to provide a device which allows to measure subjective convergence, i.e., the pupillary distance that occurs when the eyes look at near objects.

Another object is to provide a device whose adjustments can be performed even directly by the user.

Another object is to provide an instrument which has a simple structure but is capable of precisely measuring subjective near pupillary distance.

Another object is to provide an instrument which is also capable of measuring subjective interpupillary distance range and the corresponding height.

This aim and these and other objects which will become better apparent hereinafter are achieved by a device particularly for measuring subjective convergence, characterized in that it comprises a device frame, which can be coupled to the frame of a pair of eyeglasses, and supports two elements which can each be subjected to a horizontal translational motion by the user at a lens, on a plane which is parallel to the plane of arrangement of the lenses, each one of said movable elements comprising: a first portion for kinematic connection to the device frame; a second portion, which corresponds to the upper portion of the lens and is provided with at least one row of first reference marks, for the corresponding pupil of the user, said marks being arranged in vertical succession; and a third portion, which corresponds to the lower portion of the lens, being provided with a second reference mark, for the corresponding pupil of the user, and suitable to form a sight, the translational motions of said movable elements being detectable by means of a graduated scale of the device frame.

Further characteristics and advantages of the invention will become better apparent from the description of a preferred but not exclusive embodiment thereof, illustrated only by way of non-limitative example in the accompanying drawings, wherein:

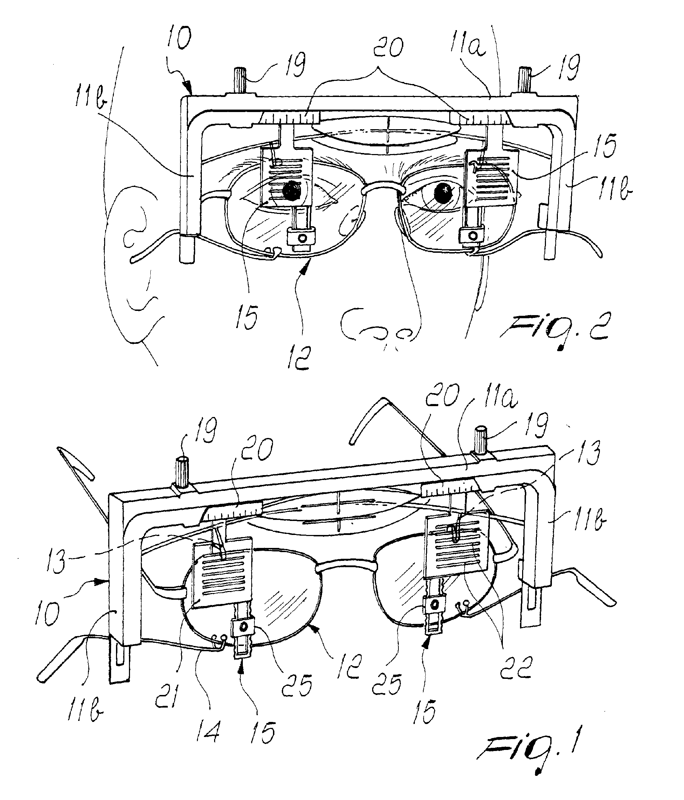

Figure 1 is a view of a device according to the invention, associated with a pair of eyeglasses;
Figure 2 is a view of a device according to the invention, associated with a pair of eyeglasses worn by a user;
Figure 3 is a front view of a portion of the device according to the invention, associated with a pair of eyeglasses;

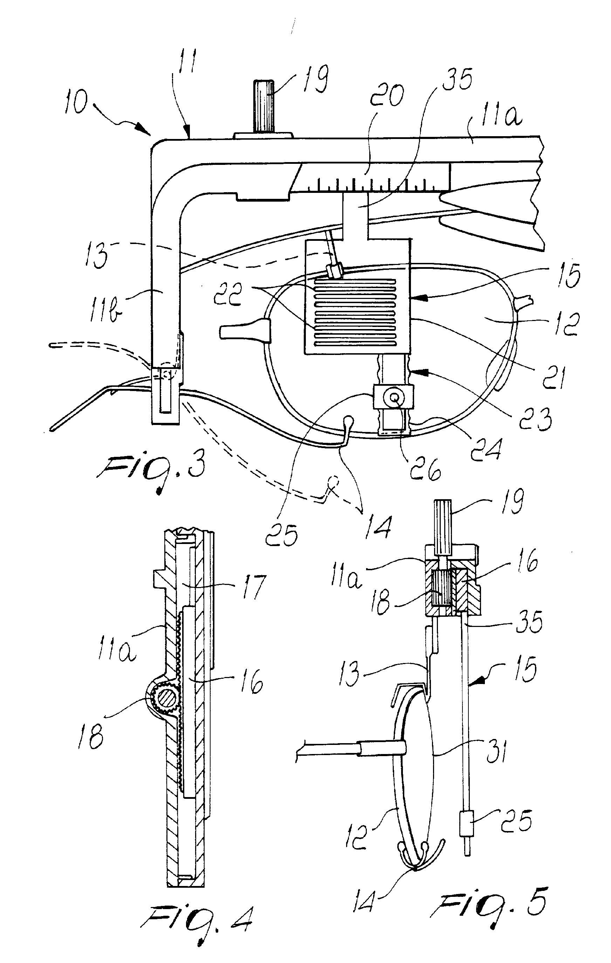

Figures 4 and 5 are two different partially sectional views of a portion of the device;

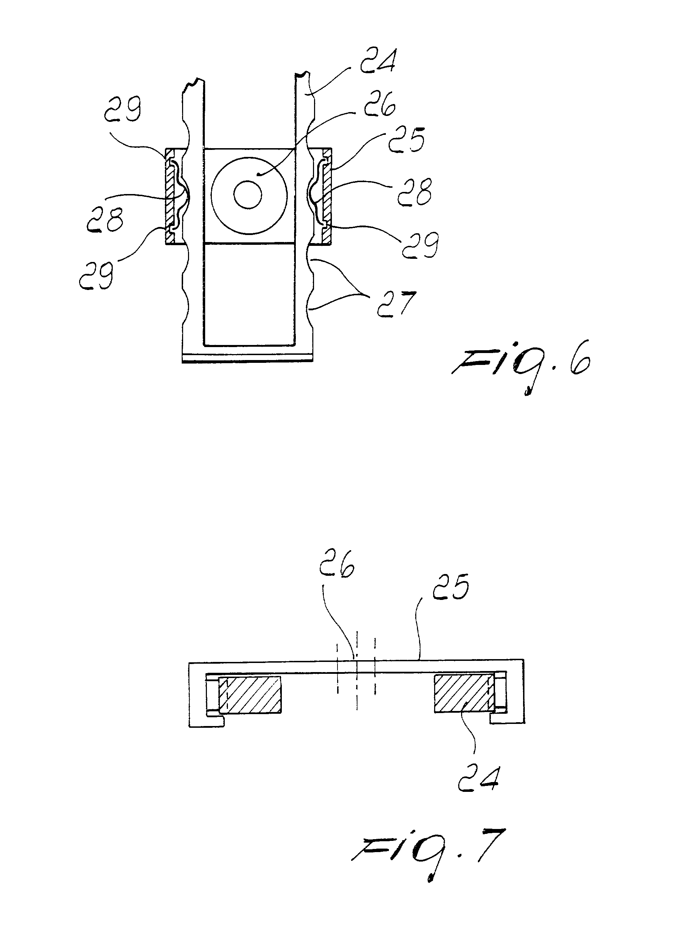

Figures 6 and 7 are two different partially sectional views of a detail of the device;
Figure 8 is a view of another component of the device;
Figure 9 is a view of a progressive-addition lens;
Figures 10 and 11 are schematic views of two steps of the operation of the device according to the invention.

With reference to the figures, a device particularly for measuring subjective convergence is generally designated by the reference numeral 10 in one of its possible embodiments.

The device 10 is constituted by a frame 11 which is shaped like an inverted U and is constituted by a longitudinal member 11a from the ends of which respective posts 11b protrude.

The device frame 11 can be attached to a pair of eyeglasses, generally designated by the reference numeral 12, by way of fork-like fixed locators 13 which are arranged opposite movable fork-like locators 14 which are articulated to the lower portions of the posts 11b and grip the frame of the eyeglasses 12 respectively from above and from below.

The device frame 11 supports two elements 15 which can perform a translational motion parallel to the longitudinal member 11a.

The movable elements 15 are substantially plate-like and are made of transparent plastics.

The ability to perform a translational motion is determined by the coupling between a rack 16, to which each element is coupled, and a corresponding guide 17 provided in the longitudinal member 11a.

Each rack 16 engages a corresponding pinion 18 which is rotatably coupled to the longitudinal member 11a and is rigidly coupled to a respective knob 19 arranged above the device frame 11 and which can be operated by the user.

Each movable element 15 moves at a graduated scale 20 which is arranged on the longitudinal member 11a of the device frame 11.

The movable elements 15 are constituted by a plate which has a first upper portion 35 for connection to the rack 16 and a second central portion 21 which corresponds to the upper portion of the lenses of the eyeglasses 12, said lenses being provided with a row of slotted holes 22, each arranged along the direction of the translational motion of said movable element, i.e., parallel to the longitudinal member 11a, and in vertical succession so as to form a grid.

The third lower portion 23 of the movable elements 15 is substantially constituted by a vertical guide 24 on which a sliding block 25 slides; said sliding block is provided with a central hole 26 which is ringed and colored so as to form a sight.

Along the guide 24, the sliding block 25 finds a plurality of positions of equilibrium, since the vertical guide 24 is shaped externally so as to define locator recesses 27 for corresponding elastic elements, advantageously leaf springs 28, which are mounted on corresponding seats 29 of the sliding block 25.

As regards operation, the device 10 is attached to a pair of eyeglasses 12 worn by a user.

While one eye is covered, as shown in Figure 11, the user looks at a reference line 30, which advantageously can be the edge of the binding of the pages of an open book.

At this point, by acting on the knob 19 that corresponds to the movable element 15 of the open eye, the user moves said element 15 until the ringed hole 26 of the sliding block 25 becomes interposed between the pupil and the reference line 30.

The next step consists in covering the previously uncovered eye and in repeating the same operation with the second eye.

At the end of this second step, the user, while both of his eyes are uncovered, looks at the reference line 30, and if the operations have been performed correctly the two ringed holes 26 of the movable elements 15 should overlap.

Advantageously, the ringed holes 26 of each movable element 15 are provided in different colors, for example red and blue, so that they can be easily distinguished by the user.

In some rare cases the two ringed holes 26 might not overlap but be offset along the vertical reference line 30.

At this point, this misalignment is balanced by acting on the sliding blocks 25 until both ringed holes 26 overlap.

Once the ringed holes 26 overlap, the near pupillary distance, i.e., the individual convergence of the user, is measured by way of the graduated scale 20.

At this point, the user is asked to look at a point of the horizon, and besides being able to measure the interpupillary distance range, by means of the slotted holes 22 provided in the movable elements 15 it is possible to determine the height at which the pupils lie when they look at an object located at a distance.

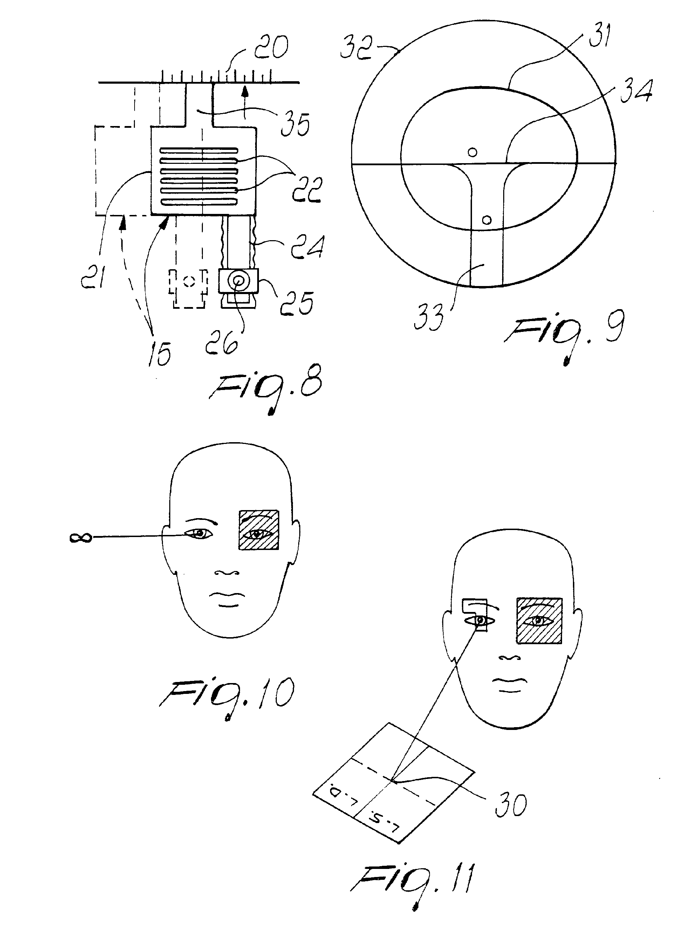

The values obtained from the preceding measurements, i.e. in particular subjective convergence, pupillary distance and the height of the pupils when the user looks at infinity, allow to determine, as shown in Figure 9, the correct position of the lens 31 to be cut on the initial lens 32.

The parameters obtained above are fundamental in determining the horizontal position of the central corridor 33 with respect to the lens 31, through which the pupil is able to focus near objects, and the imaginary line 34 that divides the lens 31 from the upper portion, through which the pupil can focus distant elements and the lower portion, particularly the central corridor.

In practice it has been observed that the present invention has achieved the intended aim and objects.

In particular, it is evident that the traditional method of determining the type of bifocal or progressive-addition lens for each user has been brought to a complete change.

Starting from the determination of subjective convergence one is in fact certain to be able to provide a pair of multifocal lenses which allow the user safe and comfortable vision.

The present invention is susceptible of numerous modifications and variations, all of which are within the scope of the appended claims.

The technical details may be replaced with other equivalent elements; the materials and the dimensions may further be any according to requirements.

The disclosures in Italian Patent Application no. PD2000A000142, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. A device particularly for measuring subjective convergence, **characterized in that** it comprises a device frame, which can be coupled to the frame of a pair of eyeglasses, and supports two elements which can each be subjected to a horizontal translational motion by the user at a lens on a plane which is parallel to the plane of arrangement of the lenses, each one of said movable elements comprising: a first portion for kinematic connection to the device frame; a second portion, which corresponds to the upper portion of the lens and is provided with at least one row of first reference marks, for the corresponding pupil of the user, said marks being arranged in vertical succession; and a third portion, which corresponds to the lower portion of the lens, being provided with a second reference mark for the corresponding pupil of the user, and suitable to form a sight, the translational motions of said movable elements being detectable by means of a graduated scale of the device frame.

2. The device according to claim 1, **characterized in that** each movable element is a plate made of transparent material.

3. The device according to claims 1 and 2, **characterized in that** said first reference marks of the second portion of the movable element are constituted by a row of slotted holes, each of which lies along the direction of the translational motion of the movable element, said holes being arranged vertically so as to form a grid.

4. The device according to claims 1 and 2, **characterized in that** said third portion of the movable element is constituted by a vertically arranged guide for a sliding block on which said second reference mark, suitable to form a sight, is provided.

5. The device according to claim 4, **characterized in that** said sight is constituted by a colored ringed hole.

6. The device according to claims 4 and 5, **characterized in that** said guide is externally provided with a plurality of pairs of opposite recesses for corresponding elastic locator elements which are associated with the sliding block.

7. The device according to claim 5, **characterized in that** said ringed holes, one for each movable element, have different colors.

8. The device according to claim 1, **characterized in that** each movable element is rigidly coupled to a rack which can slide within a corresponding guide of the frame and is coupled to a pinion rotatably coupled to the device frame and can be moved by the user.

9. The device according to claim 8, **characterized in that** said pinion is rigidly coupled to a knob.
